# EUROPEAN PATENT APPLICATION

(11) **EP 4 005 593 A1**
(43) Date of publication of application: **01.06.2022**
(21) Application number: 20843953.9
(22) Date of filing: 22.07.2020
(51) Int. Cl.: A61K 39/395, C07K 16/28, A61P 35/00

(54) **MULTIVARIABLE DOSING METHOD FOR USE IN TREATING HIGH-EGFR EXPRESSION CANCER**

(30) Priority: 24.07.2019 CN 201910673607
(71) Applicant: Sinocelltech Ltd., Beijing 100176 (CN)
(72) Inventor: XIE, Liangzhi, Beijing 100176 (CN); SU, Yahui, Beijing 100176 (CN); WANG, Yan, Beijing 100176 (CN); QIAO, Foxiao, Beijing 100176 (CN); LI, Wei, Beijing 100176 (CN); LIU, Xisheng, Beijing 100176 (CN); CHEN, Weiqiu, Beijing 100176 (CN)
(74) Representative: Simpson, Kirsty Mairi
(86) International application number: PCT/CN2020/103618
(87) International publication number: WO 2021/013207

(57) **Abstract**

The present disclosure relates to a multiple-variable dosage regimen for the treatment of cancers with high expression of EGFR. Particularly, the present disclosure relates to a multiple-variable dosage regimen for treating cancers with high EGFR expression in a subject in need, comprising: administering a first dose of anti-EGFR antibody or antigen-binding fragment thereof to said subject in a first treatment cycle; and subsequently administering a second dose of anti-EGFR antibody or antigen-binding fragment thereof to said subject during the second treatment cycle. The regimen described in the present disclosure realizes favorable effects to cancer treatment.

## Description

### RELATED APPLICATION

This application relates to Chinese Patent No. CN101058609B filed on May 26, 2006 and granted on April 13, 2011, the content of which is hereby incorporated by reference in its entirety.

### TECHNICAL FIELD

The present disclosure relates to a multiple-variable dosage regimen for the treatment of cancers with high expression of EGFR, and methods for using the regimen to treat and prevent EGFR-related diseases.

### BACKGROUND

EGFR is a member of the erbB family, which includes 4 relevant cell membrane receptors, erbB1 (EGFR, Her1), erbB2 (Her2), erbB3 (Her3) and erbB4 (Her4). EGFR is a transmembrane glycoprotein comprising an extracellular ligand-binding region and an intracellular tyrosine kinase activity region having signal transduction function. EGFR can form homodimers with ligands or form heterodimers with family members. The formation of dimers can further activate cell signal transduction pathways and regulate cell proliferation, differentiation, migration, invasion and angiogenesis (see Schlessinger J. Ligand-induced, receptor-mediated dimerization and activation of EGF receptor. Cell 2002;110 :669-72; Sako Y, Minoghchi S, Yanagida T. Single-molecule imaging of EGFR signaling on the surface of living cells. Nat Cell Biol 2000; 2:168-72; or Schlessinger J. Cell signaling by receptor tyrosine kinases. Cell 2000;103: 211-25).

Dysregulation of EGFR function or expression has been observed in common cancers (such as lung cancer, colon cancer, head and neck cancer) and non-epithelial malignancies (such as gliocytoma). These malignancies are usually associated with poor prognosis of patients (Mendelsohn (2002) J. Clin. Oncol. 20:1S-13S). A number of studies have confirmed that EGFR is highly expressed in multiple solid tumors such as head and neck squamous cell carcinoma, colorectal cancer, and esophageal squamous cell carcinoma, suggesting that the EGFR signaling pathway plays an important role in the occurrence and development of these tumors. Anti-EGFR monoclonal antibodies specifically bind to EGFR, thereby blocking the activation of EGFR signaling pathway and realizing the purpose of tumor inhibition. Up to now, multiple anti-EGFR monoclonal antibodies (cetuximab, panitumumab, etc.) have been approved both domestically and abroad for the treatment of multiple indications, including colorectal cancer, head and neck squamous cell carcinoma, and lung cancer.

There is a need for the treatment of cancers with high EGFR expression in a safe and effective manner. The present disclosure relates to a multiple-variable dosage regimen for the treatment of cancers with high expression of EGFR, and methods for using the regimen to treat and prevent EGFR-related diseases.

### SUMMARY

In one aspect, the present disclosure provides a multiple-variable dosage regimenfor treating cancers with high EGFR expression in a subject in need, comprising:
Administering a first dose of anti-EGFR antibody or antigen-binding fragment thereof to said subject during the first treatment cycle; and
Subsequently, administering a second dose of anti-EGFR antibody or antigen-binding fragment thereof to said subject during the second treatment cycle.

In one embodiment, said first treatment cycle is the first 1-6 weeks of treatment.

In one embodiment, said first dose is administered once a week during the first treatment cycle.

In one embodiment, said first dose is 6 mg/kg to 12 mg/kg, preferably 6 mg/kg, 9 mg/kg or 12 mg/kg, most preferably 6 mg/kg.

In one embodiment, said second treatment cycle is a second treatm ent cycle subsequent to the first treatment cycle. In some embodiments, sai d second treatment cycle is also referred to as the maintenance treatment sta ge.

In one embodiment, said second dose is administered every two weeks during the second treatment cycle.

In one embodiment, said second dose is 8 mg/kg to 15 mg/kg, preferably 8 mg/kg, 12 mg/kg or 15 mg/kg, most preferably 8 mg/kg.

In one embodiment, the multiple-variable dosage regimen can also be combined with other regimen.

In one embodiment, said cancer with high EGFR expression is colorectal cancer, head and neck cancer, lung cancer, breast cancer, esophageal cancer, gastric cancer, intestinal cancer, gastroesophageal junction cancer, liver cancer, pancreatic cancer, cholangiocarcinoma, gallbladder carcinoma, kidney cancer, or ovarian cancer.

In one embodiment, said subject suffers from metastatic colorectal cancer and has failed treatment with fluorouracil, oxaliplatin, and irinotecan;

Wherein, the multiple-variable dosage regimen includes dose of 6 mg/kg once a week for the first 6 weeks; subsequent dose of 8 mg/kg once every two weeks for maintenance treatment until disease progression or intolerable toxicity occurs.

In one embodiment, said subject suffers from recurrent and/or metastatic head and neck squamous cell carcinoma and has failed platinum-based drugs treatment;

Wherein, the multiple-variable dosage regimen includes dose of 6 mg/kg once a week for the first 6 weeks; subsequent dose of 8 mg/kg once every two weeks for maintenance treatment until disease progression or intolerable toxicity occurs.

In one embodiment, said subject suffers from recurrent and/or metastatic head and neck squamous cell carcinoma and has not received systemic treatment previously;

Wherein, the multiple-variable dosage regimen includes dose of 6 mg/kg once a week for the first 6 weeks; subsequent dose of 8 mg/kg once every two weeks for maintenance treatment;

Wherein, the multiple-variable dosage regimen is combined with the PF regimen, wherein said PF regimen is a cisplatin combined 5-fluorouracil treatment regimen, and the cisplatin dosage regimen is dosing on the first day with a dose of 75mg/m²; the 5-fluorouracil dosage regimen is dosing for 5 consecutive days with a dose of 750mg/m²;

Wherein, the total dosing cycle is up to 6 cycles of treatment wherein 3 weeks is 1 cycle, until disease progression or intolerable toxicity occurs.

In one embodiment, said subject suffers from recurrent or metastatic triple-negative breast cancer that has received at least first-line systemic treatment previously;
Wherein, the multiple-variable dosage regimen includes dose of 6 mg/kg once a week for the first 6 weeks; subsequent dose of 8 mg/kg once every two weeks for maintenance treatment until disease progression or intolerable toxicity occurs.

In one embodiment, said subject suffers from advanced esophageal squamous cell carcinoma that has failed platinum-based, taxane-based, or fluorouracil-based therapy previously;
Wherein, the multiple-variable dosage regimen includes dose of 6 mg/kg once a week for the first 6 weeks; subsequent dose of 8 mg/kg once every two weeks for maintenance treatment until disease progression or intolerable toxicity occurs.

In one embodiment, said subject suffers from locally advanced or metastatic squamous non-small cell lung cancer that has failed treatment with at least two chemotherapy regimens previously;
Wherein, the multiple-variable dosage regimen includes: dose of 9 mg/kg once a week for the first 6 weeks; subsequent dose of 12 mg/kg once every two weeks for maintenance treatment until disease progression or intolerable toxicity; or dose of 12mg/kg once a week for the first 6 weeks; subsequent dose of 15mg/kg once every two weeks for maintenance treatment until disease progression, or intolerable toxicity occurs.

In one embodiment, said subject suffers from an advanced solid tumor that has failed standard treatments previously;
Wherein, the multiple-variable dosage includes dose of 6 mg/kg once a week for the first 6 weeks; subsequent dose of 8 mg/kg once every two weeks for maintenance treatment until disease progression or intolerable toxicity occurs;
Wherein, the solid tumor is selected from one or more of gastric cancer, gastroesophageal junction cancer, liver cancer, pancreatic cancer, cholangiocarcinoma, gallbladder carcinoma, kidney cancer, and ovarian cancer.

In one embodiment, said anti-EGFR antibody or antigen-binding fragment thereof comprises a light chain variable region having a light chain CDR1 region having the amino acid sequence shown in SEQ ID NO: 1, a light chain CDR2 region having the amino acid sequence shown in SEQ ID NO: 2, and a light chain CDR3 region having the amino acid sequence shown in SEQ ID NO: 3; and a heavy chain variable region having a heavy chain CDR1 region having the amino acid sequence shown in SEQ ID NO: 4, a heavy chain CDR2 region having the amino acid sequence shown in SEQ ID NO: 5 and a heavy chain CDR3 region having the amino acid sequence shown in SEQ ID NO:6.

The regimen according to claim 17, wherein said anti-EGFR antibody or antigen-binding fragment thereof comprises a light chain variable region having the amino acid sequence shown in SEQ ID NO: 7 or the amino acid sequence having at least 90%, 92%, 95%, 98%, or 99% sequence identity therewith, and a heavy chain variable region having the amino acid sequence shown in SEQ ID NO: 8 or the amino acid sequence having at least 90%, 92%, 95%, 98%, or 99% sequence identity therewith.

In one embodiment, said anti-EGFR antibody is an IgGlK type monoclonal antibody.

In one embodiment, said anti-EGFR antibody comprises a light chain constant region having the amino acid sequence shown in SEQ ID NO: 9 or the amino acid sequence having at least 90%, 92%, 95%, 98%, or 99% sequence identity therewith., and a heavy chain constant region having the amino acid sequence shown in SEQ ID NO: 10 or the amino acid sequence having at least 90%, 92%, 95%, 98%, or 99% sequence identity therewith.

In one embodiment,, the binding affinity of said anti-EGFR antibody to the EGFR ECD recombinant protein KD = 0.5×10-10M.

In one embodiment, said anti-EGFR antibody or antigen-binding fragment thereof is N6-3 or antigen-binding fragment thereof.

### BRIEF DESCRIPTION OF THE DRAWINGS

.The present disclosure is illustrated in combination with the attached drawings, in which:
Figure 1 is a schematic diagram showing the specific binding of recombinant human anti-EGFR monoclonal antibody (N6-3), described in the Applicant's previous Chinese Patent No. CN101058609B, to the EGFR target.
Figure 2 shows the anti-tumor effects of N6-3 and cetuximab in mice.
Figure 3 shows the optimal tumor changes status (6mg/kg~15mg/kg) of N6-3 as the third-line treatment for colorectal cancer, wherein each column represents the tumor change of each patient.
Figure 4 shows the blood concentration-time curve of a single-dose of N6-3.
Figure 5 shows the blood concentration-time curve of multiple-dose of N6-3 (once a week (QW)).
Figure 6 shows the blood concentration-time curve of multiple-dose of N6-3 (once every two weeks (Q2W)).

### DETAILED DESCRIPTION

***Definitions***

Unless otherwise specified, all technical and scientific terms used herein have the meanings commonly understood by those of ordinary skill in the art to which the present disclosure belongs. For the purpose of the present disclosure, the following terms are defined to be consistent with the meanings commonly understood in the art.

As used herein, "mg/kg" refers to the dosage (mg) of the active pharmaceutical ingredient per kg of the subject's body weight.

When used herein and in the appended claims, the singular forms "a", "an", "another" and "said" include plural designations of the objects unless the context clearly dictates otherwise.

The term "multiple-variable dose" includes different doses of anti-EGFR antibodies or antigen-binding fragments thereof administered to a subject for treatment. "Multiple-variable dosage regimen" or "multiple-variable dosing method" describes a treatment regimen based on administering different amounts of anti-EGFR antibodies or antigen-binding fragments thereof at different time points throughout the course of treatment. In one embodiment, the present disclosure describes a multiple-variable dosage regimen comprising a first treatment cycle and a second treatment cycle, wherein the second treatment cycle is administered at a higher dose and at greater time intervals. In one embodiment, the first treatment cycle refers to the time period from the start of treatment, preferably the first 1-6 weeks from the start of treatment. The second treatment cycle is the treatment cycle subsequent to the first treatment cycle. The duration of the second treatment cycle is not particularly limited. The second treatment cycle can be from the end of the first treatment cycle to 12 months of treatment, disease progression occurs, or intolerable toxicity occurs. In some embodiments, the second treatment cycle is also referred to as the maintenance treatment stage.

The term "antibody" means an immunoglobulin molecule, and refers to any form of antibody that exhibits the desired biological activity, including but not limited to monoclonal antibodies (including full-length monoclonal antibodies), polyclonal antibodies and multi-specific antibodies (such as bispecific antibodies), and even antibody fragments. Typically, the full-length antibody structure preferably comprises 4 polypeptide chains, usually 2 heavy (H) chains and 2 light (L) chains associated to each other by disulfide bonds. Each heavy chain contains a heavy chain variable region and a heavy chain constant region. Each light chain contains a light chain variable region and a light chain constant region. In addition to this typical full-length antibody structure, its structure also includes other derivative forms.

Said heavy chain variable region and light chain variable region can be further subdivided into more conservative regions (called framework regions (FR)) and hypervariable regions (called complementarity determining regions (CDR)) interspersed therewith.

The term "complementarity determining region" (CDR, such as CDR1, CDR2, and CDR3) refers to such amino acid residues of the variable region of an antibody, the presence of which is necessary for antigen binding. Each variable region typically has 3 CDR regions identified as CDR1, CDR2, and CDR3. Each complementarity determining region may contain amino acid residues from the "complementarity determining region" defined by Kabat (Kabat et al., Sequences of Proteins of Immulological Interest, 5th Ed. Public Health Service, National Institutes of Health, Bethesda, MD. 1991 )) and/or those residues from the "hypervariable loop" (Chothia and Lesk; J Mol Biol 196: 901-917 (1987)).

Each heavy chain variable region and light chain variable region usually comprises 3 CDRs and up to 4 FRs. Said CDRs and FRs are arranged from the amino terminus to the carboxyl terminus in the following order, for example: FR1, CDR1, FR2, CDR2, FR3, CDR3, FR4.

The complementarity determining regions (CDRs) and framework regions (FRs) of a given antibody can be identified using the Kabat system (Kabat et al.: Sequences of Proteins of Immunological Interest, 5th edition, US Department of Health and Human Services, PHS, NIH, NIH publication No. 91-3242, 1991).

The term "constant region" refers to such amino acid sequences on the light chain and heavy chain of an antibody, which do not directly participate in the binding of the antibody to the antigen, but exhibit a variety of effector functions, such as antibody-dependent cell-mediated cytotoxicity.

On the ground of the antigenic differences of the amino acid sequence of the constant region, the heavy chain of an antibody can be classified into five classes: α, δ, ε, γ, and µ; when forming a complete antibody with the light chain, it can be classified into five classes: IgA , IgD, IgE, IgG and IgM, several of these classes can be further classified into subclasses (isotypes), such as IgG1, IgG2, IgG3, IgG4, IgA and IgA2. On the ground of the amino acid sequence of the constant region, the light chain of an antibody can be classified into kappa and lambda.

An "antigen-binding fragment of an antibody" comprises a portion of a complete antibody molecule, that retains at least some of the binding specificity of the parent antibody, and usually comprises at least a portion of the antigen-binding region or variable region (such as one or more CDRs) of the parent antibody. Examples of antigen-binding fragments include, but are not limited to, Fv, Fab, Fab', Fab'-SH, F(ab')2, Fd fragment, Fd' fragment, single-chain antibody molecules (e.g., scFv, di-scFv, or tri-scFv , Diabody or scFab), single domain antibody.

The term "antibody fragment" refers to a non-complete antibody molecule that retains at least some biological properties of the parent antibody, and examples thereof include, but are not limited to, Fc fragments in addition to those mentioned in the aforementioned "antigen-binding fragments".

The term "chimeric antibody" refers to an antibody in which a part of the heavy chain and/or light chain is derived from a specific source or species, and the remaining part is derived from a different source or species. The "chimeric antibody" may also be a functional fragment as defined above. "Humanized antibody" is a subset of "chimeric antibody".

The term "humanized antibody" or "humanized antigen-binding fragment" is defined herein as an antibody or antibody fragment that is: (i) derived from a non-human source (for example, a transgenic mouse carrying a heterologous immune system) and based on the human germline sequence; or (ii) chimeric antibodies in which the variable region is of non-human origin and the constant region is of human origin; or (iii) CDR transplants in which the CDRs in the variable region are of non-human origin and one or more of the FRs in the variable region is of human origin and the constant region (if any) is of human origin. The purpose of "humanization" is to eliminate the immunogenicity of antibodies of non-human origin in humans, while retaining the maximum possible affinity. It is advantageous to select a human frame sequence that is most similar to the frame sequence of a non-human source antibody as a template for humanization. In some cases, it may be necessary to replace one or more amino acids in the human frame sequence with corresponding residues in the non-human frame to avoid loss of affinity.

A "monoclonal antibody" refers to an antibody obtained from a substantially homogeneous antibody population, i.e., said population comprising identical antibodies except for possible mutations (such as natural mutations) that may be present in very small amounts. Thus, the term "monoclonal" indicates the properties of said antibodies, that is, not a mixture of unrelated antibodies. In contrast to polyclonal antibody formulations, which usually include different antibodies directed against different determinants (epitopes), each monoclonal antibody of a monoclonal antibody formulation is directed against one single determinant on the antigen. In addition to their specificity, the advantage of monoclonal antibody formulations is that they are generally not impurified by other antibodies. The term "monoclonal" should not be understood as requiring any particular method of producing said antibodies.

The antibody "specifically binds" to a target antigen such as a tumor-associated peptide antigen target, i.e. binds said antigen with sufficient affinity to enable said antibody to be used as a therapeutic reagent, targeting a cell or tissue expressing said antigen, and does not significantly cross-react with other proteins , or does not significantly cross-react with proteins other than the homologues and variants of the target proteins mentioned above (e.g. mutant forms, splice variants, or protein hydrolysis truncated forms).

The term "binding affinity" refers to the strength of the sum of the non-covalent interactions between a molecule's individual binding sites and its binding partners. Unless otherwise stated, "binding affinity", when used herein, refers to the intrinsic binding affinity, which reflects a 1:1 interaction between members of a binding pair (e.g. antibody and antigen). As used herein, the term "KD" refers to the equilibrium dissociation constant of the antibody-antigen interaction. As used herein, the term "kₒₙ" refers to the rate constant at which an antibody binds to an antigen. As used herein, the term "k_{off}" refers to the rate constant at which an antibody dissociates from an antibody/antigen complex. "KD", "binding rate constant kₒₙ" and "dissociation rate constant k_{off}" are commonly used to describe the affinity between a molecule (e.g. an antibody) and its binding partner (e.g. an antigen). Affinity, i.e. the tight degree at which a ligand binds a particular protein. Binding affinity is influenced by non-covalent intermolecular interactions such as hydrogen bonding, electrostatic interactions, hydrophobic and van der Waals forces between two molecules. In addition, the binding affinity between a ligand and its target molecule may be influenced by the presence of other molecules. Affinity can be analyzed by conventional methods known in the art, including ELISA described herein.

The term "epitope" includes any protein determinant capable of specifically binding to an antibody or T cell receptor. Epitope determinants typically consist of chemically active surface groups of molecules (such as amino acids or sugar side chains, or combinations thereof), and usually have specific three-dimensional structural characteristics and specific charge characteristics.

The term "isolated" antibody is an antibody that has been identified and isolated from the component of the cell in which it is expressed. Isolated antibodies include in situ antibodies in recombinant cells, where at least one component of the natural environment of said antibodies is not present. However, isolated antibodies are typically prepared by at least one purification step.

The term "dose" refers to the amount of EGFR antibody administered to a subject.

The term "multiple-variable dose" includes different doses of EGFR antibody administered to a subject for treatment. "Multiple-variable dosage regimen" or "multiple-variable dosage therapy" describes a treatment regimen based on administering different amounts of EGFR antibody at different time points throughout the course of treatment. In one embodiment, the invention disclosure describes a multiple-variable dosage regimen including a first treatment cycle and a second treatment cycle, wherein a higher dose of EGFR antibody is administered during the second treatment cycle.

The term "combination" includes the co-administration of a first active pharmaceutical ingredient and a second active pharmaceutical ingredient, for example, they can be dissolved or mixed into the same pharmaceutically acceptable carrier, or the first active pharmaceutical ingredient can be administered followed by the second active pharmaceutical ingredient, or the second active pharmaceutical ingredient can be administered followed by the first active pharmaceutical ingredient. Thus, the present disclosure includes combined treatment methods and combined pharmaceutical compositions.

The term "combination therapy" refers to the administration of two or more therapeutic substances, such as an anti-EGFR antibody and another one or more drugs. The administration of the anti-EGFR antibody is accompanied by the administration of the other drug(s) at the same time, before or after the administration of the anti-EGFR antibody.

### The antibody and antigen-binding fragment thereof in the present disclosure

Said anti-EGFR antibody or antigen-binding fragment thereof described in the present disclosure can be any anti-EGFR antibody or antigen-binding fragment thereof, preferably the anti-EGFR antibody or antigen-binding fragment thereof described in the Applicant's previous Chinese Patent No. CN101058609B.

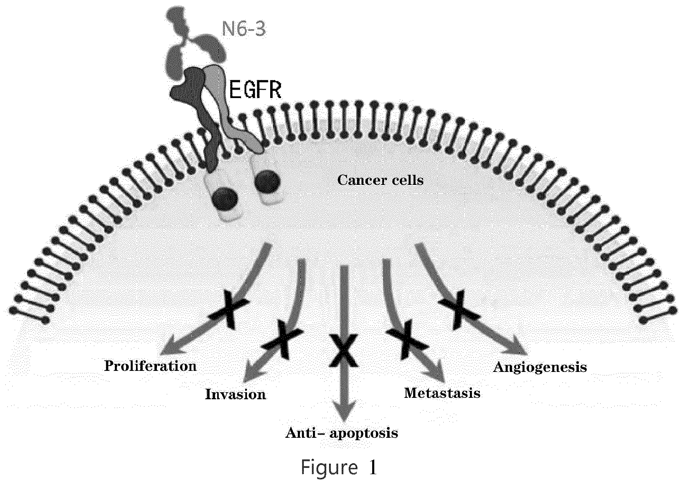

The recombinant fully human anti-EGFR monoclonal antibody N6-3 described in the Applicant's previous Chinese Patent No. CN101058609B is an IgGlK type monoclonal antibody composed of 1326 amino acids and with a molecular weight of about 145kDa. N6-3 can specifically bind to the extracellular ligand binding region of the EGFR target and become a competitive antagonist of endogenous ligands (see Figure 1).

N6-3 simultaneously exerts its anti-tumor efficacy through EGFR antagonism and antibody-dependent cell-mediated cytotoxicity. As a fully human IgG1 type anti-EGFR antibody, the antigen binding epitope, the physical and chemical properties, and biological activities of N6-3 are different from those of the marketed drugs.

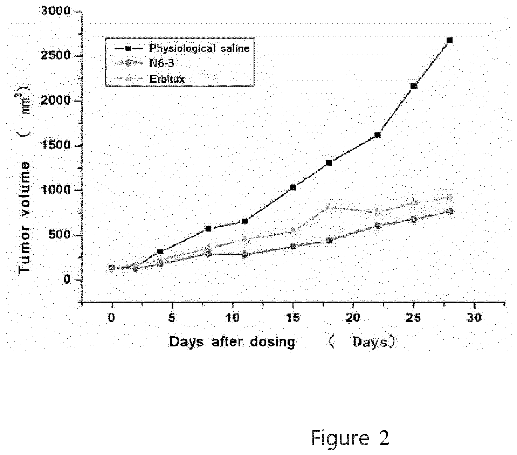

The Inventors found that in pre-clinical studies, N6-3 exhibited better anti-tumor effects than cetuximab (Erbitux) (see Figure 2). The results of a phase I clinical study of N6-3 monotherapy for advanced colorectal cancer suggest that its initial efficacy is significantly better than that of cetuximab. Combined with the pharmacokinetic data, the Inventors determined said multiple-variable dosage regimen described herein.

In one aspect, said antibody described herein is the antibody N6-3 described in the Applicant's previous Chinese Patent No. CN101058609B. The binding affinity of the antibody to the EGFR ECD recombinant protein KD=0.5×10⁻¹⁰M.

See Table 1 for the related amino acid sequences of antibody N6-3.

**Table 1. Amino acid sequence of antibody N6-3**

| Sequence identity | Amino acid sequence | Sequence No. |
|---|---|---|
| L-CDR1 | QDISNYLN | SEQ ID NO : 1 |
| L-CDR2 | DASSLET | SEQ ID NO : 2 |
| L-CDR3 | QHFDHLPLA | SEQ ID NO : 3 |
| H-CDR1 | GGSVSSGDYYWS | SEQ ID NO : 4 |
| H-CDR2 | HIYYSGNTNYNPSLKS | SEQ ID NO : 5 |
| H-CDR3 | VRDRVTGAFDI | SEQ ID NO : 6 |
| Light chain Variable region | DIVMTQSPSTLSASVGDRVTITCQ ASQDISNYLNWYQQKPGKAPKLLI YDASSLETGVPSRFSGSGSGTDFTF TISGLQPEDIATYFCQHFDHLPLAF GGGTKVEIKR | SEQ ID NO : 7 |
| Heavy chain variable region | QVQLQESGPGLVKPSETLSLTCTVSG GSVSSGDYYWSWIRQSPGKGLEWI GHIYYSGNTNYNPSLKSRLTISIDTSK NQFSLKLSSVTAADTAIYYCVRDRV TGAFDIWGQGTMVTVSS | SEQ ID NO : 8 |
| Light chain constant region | TVAAPSVFIFPPSDEQLKSGTASVVC LLNNFYPREAKVQWKVDNALQSGN SQESVTEQDSKDSTYSLSSTLTLSKA DYEKHKVYACEVTHQGLSSPVTKSF NRGEC | SEQ ID NO : 9 |
| Heavy chain constant region | | SEQ ID NO : 10 |
| | | |

### Uses

The multiple-variable dosage regimen of the present disclosure can be used to treat cancers with high EGFR expression, including colorectal cancer, head and neck cancer (e.g., head and neck squamous cell carcinoma), lung cancer (e.g., small cell lung cancer or non-small cell lung cancer, such as squamous non-small cell lung cancer), breast cancer, esophageal cancer (e.g., esophageal squamous cell carcinoma), gastric cancer, bowel cancer, gastroesophageal junction cancer, liver cancer, pancreatic cancer, cholangiocarcinoma, gallbladder carcinoma, kidney cancer, or ovarian cancer.

The term "treatment" used in the present disclosure generally refers to obtaining a desired pharmacological and/or physiological effect. The effect may be prophylactic in terms of completely or partially preventing the disease or its symptoms; and/or may be therapeutic in terms of partially or completely stabilizing or curing the disease and/or side effects due to the disease. "Treatment" as used herein covers any treatment of a patient's disease, including: (a) preventing diseases or symptoms in patients who are susceptible to diseases or symptoms but have not yet been diagnosed with the disease; (b) suppressing disease symptoms, i.e., stopping its progression; or (c) relieving the symptoms of the disease, i.e., causing the disease or symptoms to degenerate.

### Pharmaceutical compositions

A pharmaceutical composition comprising the said anti-EGFR antibody or antigen-binding fragment thereof can be prepared, comprises said anti-EGFR antibody or antigen-binding fragment thereof and one or more pharmaceutically acceptable carriers.

Methods for preparing various pharmaceutical compositions containing various ratios of active ingredients are known or are apparent to those skilled in the art in accordance with the disclosure of the present application. As described in REMINGTON'S PHARMACEUTICAL SCIENCES, Martin, E.W., ed., Mack Publishing Company, 19th ed. (1995). The method for preparing said pharmaceutical composition includes incorporating appropriate pharmaceutical excipients, carriers, diluents and the like. Said pharmaceutical composition described in the present disclosure is produced by known methods, including conventional methods of mixing, dissolving or lyophilisation.

In said pharmaceutical compositions described in the present disclosure, the proportion of active ingredients may vary, and may range from about 0.01% to about 99% of the weight of a given dosage form. In such therapeutically useful formulations of pharmaceutical compositions, the amount of the active ingredient allows for an effective dose level to be obtained.

The active ingredient can also be administered intravenously or intraperitoneally by infusion or injection. A buffered aqueous solution of the active ingredient can be prepared, optionally mixed with an osmotic pressure regulating agent, stabilizing agent and surfactant.

The therapeutically effective amount of the active ingredient depends on, in addition to the property of the active ingredient itself, the mode of administration, the nature of the disease to be treated, and the age and health status of the patient, and ultimately depends on the decision of the physician or clinician on site.

The aforementioned formulations may be presented in a dosage form, which is a physically discrete unit containing a unit dose, suitable for administration to humans and other mammals.

The following examples facilitate a better understanding of the present disclosure, but do not limit the present disclosure. The experimental methods in the following examples are conventional methods unless otherwise specified. The experiment materials used in the following examples, unless otherwise specified, were purchased from conventional biochemical reagent companies.

### Example 1 Phase I clinical study of N6-3 monotherapy in the third-line treatment of colorectal cancer

### A. Study design - Phase I study of colorectal cancer

This study was designed as single-center, non-randomized, open label, dose-escalation phase I clinical trial, selecting subjects with metastatic colorectal cancer who had failed treatment with fluorouracil, oxaliplatin, and irinotecan as the study population. The trial design was consisted of a single-dose trial stage and a multiple-dose trial stage, with the subjects in single-dose stage divided into 6 dose-escalation groups (0.5mg/kg, 1.0mg/kg, 2.0mg/kg, 4.0mg/kg, 6.0mg/kg and 8.0mg/kg) for a dose-escalation trial. After a 3-week washout period, subjects who completed the single-dose tolerance assessment were entered into multiple-dose study at the original dose level, with the 0.5 mg/kg, 1.0 mg/kg, and 2.0 mg/kg dose groups receiving QW (once weekly), 4 study doses; the 4.0 mg/kg group receiving QW, 6 study doses; 6.0 mg/kg group receiving QW, 6 study doses and Q2W (once every two weeks), 3 study doses, and the 8.0 mg/kg dose group receiving Q2W, 3 study doses. Subjects who have achieved at least stable disease after completing the 6-week study treatment were entered into maintenance treatment stage until 12 months of treatment, the onset of progressive disease (PD), or intolerable toxicity occurred. After completion of the dose-escalation trial, if the target dose is determined, an extension study at the target dose is allowed.

Based on the data of the aforementioned clinical trials, on the basis of the phase I clinical research plan, three high-dose groups (9.0 mg/kg, 12.0 mg/kg, and 15.0 mg/kg) were added to the phase I clinical study protocol for a dose-escalation trial to observe the dose-limiting toxicity of N6-3.

### B. Study results - Efficacy results

Up to December 31, 2018, a total of 56 subjects were evaluated for efficacy by the investigator based on the Response Evaluation Criteria in Solid Tumors (RECIST version 1.1). The 56 subjects included 22 subjects enrolled in the dose-escalation group, 25 subjects enrolled in the 6mg/kg dose expansion group, and 9 subjects in the high-dose group added later.

The efficacy data of the dose-escalation group are shown in the table below, Among the 22 subjects, ORR was 9% (2/22) and DCR was 31.8% (7/22).

**Efficacy data of the dose-escalation group**

| mg/kg (n) | 0.5 (N=3) | 1.0 (N=3) | 2.0 (N=3) | 4.0 (N=4) | 6.0/QW (N=3) | 6.0/Q2W (N=3) | 8.0 (N=3) |
|---|---|---|---|---|---|---|---|
| CR | 0 | 0 | 0 | 0 | 0 | 0 | 0 |
| PR | 0 | 0 | 0 | 0 | 0 | 2 | 0 |
| SD | 0 | 0 | 1 | 2 | 1 | 1 | 2 |
| PD | 3 | 3 | 2 | 2 | 2 | 0 | 1 |
| ORR (%) | 0 (0%) | 0 (0%) | 0 (0%) | 0 (0%) | 0 (0%) | 2/3 (67%) | 0 (0%) |
| DCR (%) | 0 (0%) | 0 (0%) | 1/3 (33%) | 2/2 (50%) | 1/3 (33%) | 3/3 (100%) | 2/3(67%) |

| | | | | | | | |
|---|---|---|---|---|---|---|---|
| Note: CR: complete response; PR: partial response; SD: stable disease; PD: progressive disease; ORR: objective response rate, including CR and PR; DCR: disease control rate, including CR, PR, and SD. | | | | | | | |

A total of 25 subjects entered the study at the 6mg/kg/QW expansion group, and one subject withdrew from the study early. The ORR in this cohort was as high as 48% (12/25), and DCR was 88% (22/25). In contrast, the results of previous studies (ASPECCT) of cetuximab and panitumumab in third-line treatment of advanced colorectal cancer showed their ORRs were only 22% and 19%, respectively.

A total of 9 subjects in the subsequent high-dose group completed the treatment, Among them, ORR was 44.4% (4/9) and DCR was 77.8% (7/9). The detailed data are shown in the table below.

**Efficacy data of the high-dose group**

| mg/kg (n) | 9.0 (N=3) | 12.0 (N=3) | 15.0 (N=3) |
|---|---|---|---|
| CR | 0 | 0 | 0 |
| PR | 0 | 2 | 2 |
| SD | 2 | 1 | 0 |
| PD | 1 | 0 | 1 |
| ORR (%) | 0 (0%) | 2/3(67%) | 2/3(67%) |
| DCR (%) | 2/3(67%) | 3/3(100%) | 2/3(67%) |

| | | | |
|---|---|---|---|
| Note: CR: complete response; PR: partial response; SD: stable disease; PD: progressive disease; ORR: objective response rate, including CR and PR; DCR: disease control rate, including CR, PR, and SD. | | | |

For 43 subjects with a dose of 6 mg/kg and above (6 in the 6 mg/kg escalation group, 3 in the 8 mg/kg group, 9 in the high-dose group, and 25 in the 6 mg/kg expansion group), ORR was 40% (17 /43), DCR was 84% (36/43).

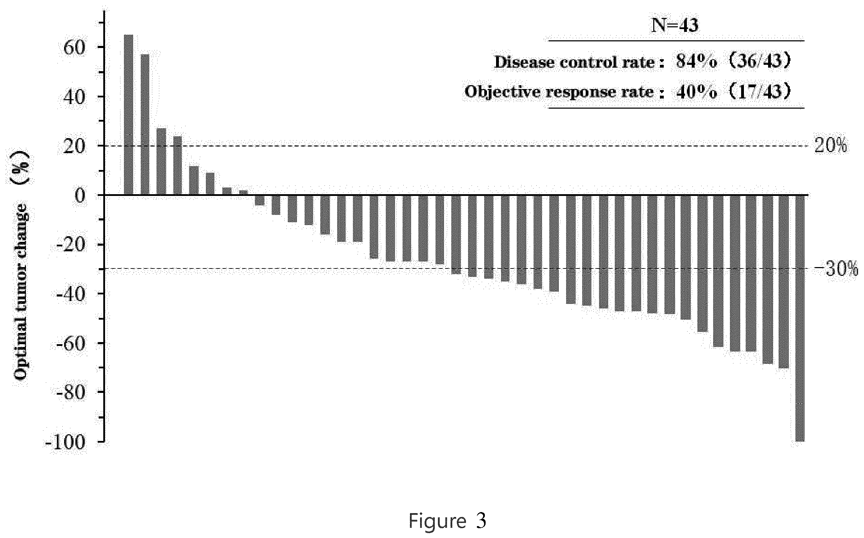

The optimal tumor changes (6mg/kg∼15mg/kg) of N6-3 third-line treatment of colorectal cancer are shown in Figure 3.

### C. Study results - Safety results

Up to August 23, 2017, based on the safety analysis results of 22 subjects in the dose-escalation group and 13 subjects in the expansion group showed that N6-3 was safe and well tolerated in subjects with advanced colorectal cancer. Neither dose-limiting toxicity nor drug-related serious adverse events were seen in the dose-escalation group (N=22); the incidence of adverse events was 100% (22/22) among which a drug-related adverse event rate of 90.9% (20/22), and 5 subjects (22.7%) had dose reduction or dose interruption due to adverse events. In the expansion group (N=13), the incidence of drug-related adverse events was 100% (13/13) among which 6 cases of dose reduction or dose interruption due to adverse events. Adverse events associated with N6-3 included cases of cutaneous toxicity, hypomagnesemia, and infusion-related reactions similar to those of special attention in the instructions of similar drugs (cetuximab and panitumumab).

### D. Study results - Pharmacokinetic results

### a. Pharmacokinetics of single dose

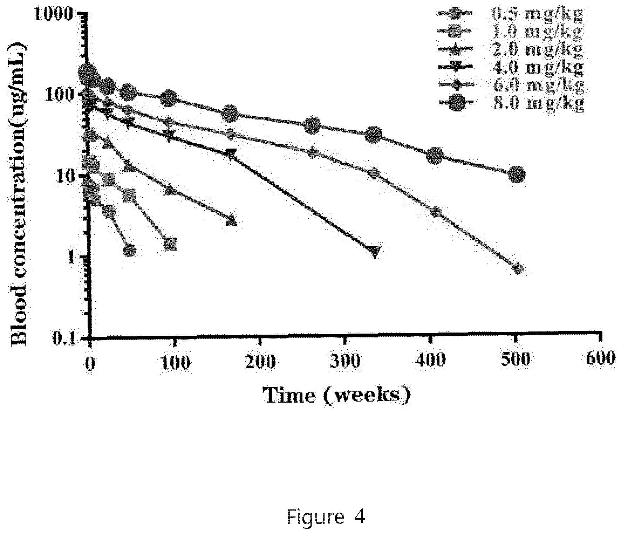

The N6-3 single-dose pharmacokinetic study was completed at 6 dose levels of 0.5mg/kg, 1.0mg/kg, 2.0mg/kg, 4.0mg/kg, 6.0mg/kg and 8.0mg/kg for evaluation. Each dose group completed a single-dose followed by a 3-week washout period and entered a multiple-dose stage based on the original dose level. After a single intravenous infusion of N6-3, the low-dose group (0.5mg/kg~2.0mg/kg) showed rapid target clearance, and the blood concentration was undetectable or close to the lower limit of detection after one week, while the high dose group (6.0mg/kg∼8.0mg/kg) at 4.0mg/kg and above showed a gradual saturation of target clearance *in vivo.* The N6-3 single-dose blood concentration-time curve is shown in Figure 4.

### b. Pharmacokinetics of multiple dose

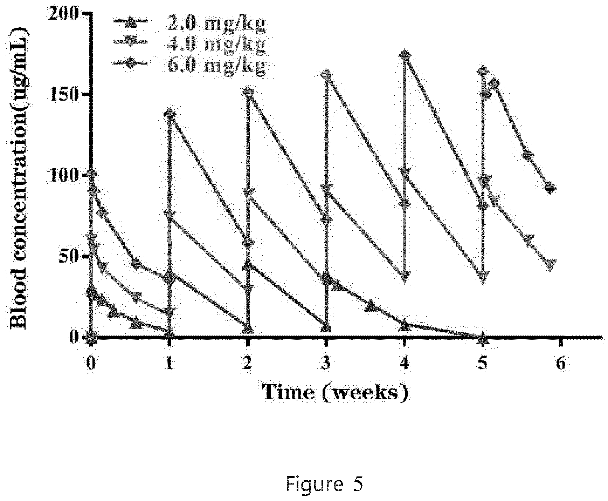

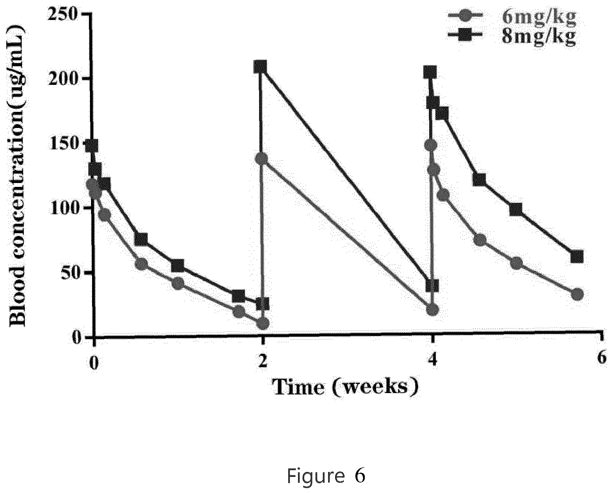

After the N6-3 once weekly (QW) groups of 4.0 mg/kg and 6.0 mg/kg reached a steady state, their trough concentrations were 43.9 µg/mL and 92.6 µg/mL, respectively, and as for the once every 2 weeks (Q2W) groups of 6.0mg/kg and 8.0mg/kg, their trough concentrations for the 3rd dose were 29.1µg/mL and 58.1µg/mL, respectively. The steady-state trough concentrations of multiple-dose of cetuximab and panitumumab were 54µg/mL and 50µg/mL, respectively. Referring to the steady-state pharmacokinetic parameters of the same target drug after clinical application, it can be inferred that, the trough concentrations could reach the saturated steady-state pharmacological activity value when N6-3 is administered at 4.0 mg/kg, 6.0 mg/kg weekly (QW) for six consecutive doses, and at 8.0 mg/kg dose every two weeks (Q2W) for three consecutive doses. The N6-3 multiple-dose (weekly dosing (QW)) blood concentration-time curve is shown in Figure 5. The N6-3 multiple-dose (biweekly dosing (Q2W)) blood concentration-time curve is shown in Figure 6.

In summary, the data from the phase I clinical study preliminarily suggest a manageable safety and tolerability profile and significant efficacy in subjects with advanced colorectal cancer treated with N6-3 as the third line treatment. Based on the N6-3 clinical phase I safety and clinical efficacy data, the Inventors determined the following phase II dosing regimen (RP2D): 6.0mg/kg/QW, for six weeks consecutive dosing; followed by tumor efficacy assessment and, for subjects in stable and response status, a subsequent dosing regimen of 8.0 mg/kg/Q2W for consecutive dosing until tumor progression or intolerable toxicity occurred.

### Example 2: Phase II clinical study of N6-3 monotherapy in the third-line treatment of colorectal cancer

**A. Study design:** The phase II clinical study was designed as multi-center, non-randomized and open label, in which 110 subjects with metastatic colorectal cancer who had failed treatment with fluorouracil, oxaliplatin, and irinotecan were selected to enter the study and receive N6-3 monotherapy (6 mg/kg administered weekly (QW) for the first 6 weeks; thereafter changed to 8 mg/kg administered biweekly (Q2W) for maintenance treatment) until disease progression, or intolerable toxicity occurred.
**B. Study progress:** Up to 30, 2019, the study had completed enrollment of 81 subjects.
**C. Study results - efficacy results:** Up to 30, 2019, a total of 55 subjects in this study have completed the study, ORR was 34.2% (26/76) and DCR was 73.7% (56/76).
**D. Study results - safety results:** Up to February 15, 2019, a total of 55 subjects in this study had at least one safety data result, as detailed in Table 2

below. Wherein, AEs (adverse events) related or possibly related to the study drug mainly include skin rash and hypomagnesemia; Grade 3 or above AEs related to the study drug include one case of anterior myocardial infarction, one case of hand-foot syndrome, and one case of acneiform eruption and 1 case of hypokalemia.

**Table 2: N6-3 interim safety results**

| | N6-3 (N=55) |
|---|---|
| Any AEs | 26 (47.3%) |
| Drug-related AEs | 24 (43.6%) |
| AEs of Grade 3 or above | 7 (12.7%) |
| Drug-related AEs of Grade 3 or above | 4 (7.3%) |

### Example 3: Phase II clinical study of N6-3 monotherapy in the second-line treatment of head and neck squamous cell carcinoma

**A. Study design:** The phase II clinical study was designed as multi-center, non-randomized and open label, in which 30-40 subjects with recurrent and/or metastatic head and neck squamous cell carcinoma who had failed treatment with platinum-based drug were selected to enter the study and receive N6-3 monotherapy (6 mg/kg administered weekly (QW) for the first 6 weeks; thereafter changed to 8 mg/kg administered biweekly (Q2W) for maintenance treatment) until disease progression, or intolerable toxicity occurred.
**B. Study progress:** Up to May 26, 2019, the study has completed enrollment of 17 subjects.
**C. Study results - efficacy results:** Up to April 9, 2019, a total of 6 subjects in this study had at least one imaging evaluation result: ORR was 50.0% (3/6), and DCR was 83.3% (5/6). Wherein, one subject had a first evaluation of complete response, and the investigator continued the study drug treatment based on the clinical benefit to the patient, and the patient remained in complete response on the 2nd subsequent tumor evaluation; the other patient had a 38.8% reduction in the target lesion on the first imaging evaluation, but the overall tumor evaluation was disease progression because of the appearance of a new lesion.

### Example 4: Phase II clinical study of N6-3+PF combination therapy in the first-line treatment of head and neck squamous cell carcinoma

**A. Study design:** The phase II clinical study was designed as multi-center, non-randomized and open label, in which 15-30 subjects with recurrent and/or metastatic head and neck squamous cell carcinoma who had not previously received systemic therapy were selected to enter the study and receive the N6-3 combined with PF regimen, in which N6-3 was administered as 6 mg/kg weekly (QW) for the first 6 weeks; thereafter, changed to 8 mg/kg administered biweekly (Q2W) for maintenance treatment; while the PF regimen was a cisplatin combined with 5-fluorouracil regimen, in which cisplatin was administered as a dose of 75 mg/m² on day 1 and 5-fluorouracil was administered as a dose of 750 mg/m² on 5 consecutive days; the total dosing cycle was 1 cycle of 3 weeks, with a maximum of 6 cycles of treatment until disease progression, or intolerable toxicity occurred.
**B. Study progress:** Up to May 26, 2019, 1 subject had been enrolled in the study.

### Example 5: Phase II clinical study of N6-3 monotherapy in the second-line and above treatment of triple-negative breast cancer

**A. Study design:** The phase II clinical study was designed as multi-center, non-randomized and open label, in which 30-40 subjects with recurrent or metastatic triple-negative breast cancer who had received at least first-line system treatment previously were selected to enter the study and receive N6- 3 monotherapy (6 mg/kg administered weekly (QW) for the first 6 weeks; thereafter changed to 8 mg/kg administered biweekly (Q2W) for maintenance treatment) until disease progression, or intolerable toxicity occurred.
**B. Study progress:** Up to May 26, 2019, 30 subjects have been enrolled in the study.
**C. Study results** - **efficacy results:** Up to May 26, 2019, 27 subjects in this study had at least 1 imaging evaluation result and the other 3 subjects fell off before first evaluation: ORR was 6.7% (2/30), DCR was 16.7% (5/30).

### Example 6: Phase Ib clinical study of N6-3 monotherapy in the second-line and above treatment of esophageal squamous cell carcinoma

**A. Study design:** The phase Ib clinical study was designed as multi-center, non-randomized and open label, in which 20-50 subjects with advanced esophageal squamous cell carcinoma who had previously failed platinum-based, taxane-based, or fluorouracil-based therapy were selected to enter the study and receive N6-3 monotherapy (6 mg/kg administered weekly (QW) for the first 6 weeks; thereafter changed to 8 mg/kg administered biweekly (Q2W) for maintenance treatment) until disease progression, or intolerable toxicity occurred.
**B. Study progress:** Up to May 26, 2019, 30 subjects had been enrolled in the study.
**C. Study results** - **efficacy results:** Up to May 26, 2019, a total of 24 subjects in this study had at least 1 imaging evaluation: ORR was 16. 7% (4/24) and DCR was 45.8% (11/24).

### Example 7: Phase Ib clinical study of N6-3 monotherapy in the third-line and above treatment of squamous non-small cell lung cancer

**A. Study design:** The phase Ib clinical study was designed as a multi-center, open clinical trial, in which subjects with locally advanced or metastatic squamous non-small cell lung cancer who had previously failed at least two chemotherapy regimens were selected to enter the study and were randomly assigned to one of two N6-3 dose study cohorts: Cohort 1 was a low-dose cohort (9 mg/kg administered weekly (QW) for the first 6 weeks; thereafter changed to 12 mg/kg administered biweekly (Q2W) for maintenance treatment), Cohort 2 was a high-dose cohort (12 mg/kg administered weekly (QW) for the first 6 weeks; thereafter changed to 15 mg/kg administered biweekly (Q2W) for maintenance treatment), 15-25 subjects were planned to be enrolled in each cohort.
**B. Study progress:** Up to May 26, 2019, 5 subjects had been enrolled in the study, of which 3 subjects were randomized to Cohort 1 and the other 2 subjects to Cohort 2. A total of 3 subjects had at least one safety data result, with no Grade 3 or above adverse events.

### Example 8: Phase Ib clinical study of N6-3 monotherapy in the treatment of advanced solid tumors that had failed standard chemotherapy

**A. Study design:** The phase Ib clinical study was designed as multi-center, non-randomized and open label, in which subjects with advanced solid tumors (gastric cancer, gastroesophageal junction cancer, liver cancer, pancreatic cancer, cholangiocarcinoma, gallbladder carcinoma, kidney cancer, ovarian cancer, etc.) who had previously failed standard chemotherapy were selected to enter the study and receive N6-3 monotherapy (6 mg/kg administered weekly (QW) for the first 6 weeks; thereafter changed to 8 mg/kg administered biweekly (Q2W) for maintenance treatment) until disease progression or intolerable toxicity occurred, 15-25 subjects were planned to be enrolled in each cohort.
**B. Study progress:** Up to May 26, 2019, 31 subjects had been enrolled in the study.
**C. Efficacy results:** Up to May 26, 2019, a total of 19 subjects in this study had at least one imaging evaluation result: ORR was 5.3% (1/19) and DCR was 42.1% (8/19).

### Equivalents

The aforementioned examples are intended to illustrate the present invention only and should not be regarded as any limitation on the scope of the present disclosure. Apparently, many modifications and changes can be made to the content described in the above embodiments and examples of the present disclosure without departing from the principle of the present disclosure. All such modifications and changes are covered by the present disclosure.

## Claims

1. A multiple-variable dosage regimen for treating cancers with high EGFR expression in a subject in need, comprising:
Administering a first dose of anti-EGFR antibody or antigen-binding fragment thereof to said subject during the first treatment cycle; and
Subsequently, administering a second dose of anti-EGFR antibody or antigen-binding fragment thereof to said subject during the second treatment cycle.

2. The regimen according to claim 1, wherein said first treatment cycle is the first 1-6 weeks of treatment.

3. The regimen according to any one of claims 1-2, wherein said first dose is administered once a week during the first treatment cycle.

4. The regimen according to any one of claims 1-3, wherein said first dose is 6 mg/kg to 12 mg/kg, preferably 6 mg/kg, 9 mg/kg or 12 mg/kg, most preferably 6 mg/kg.

5. The regimen according to any one of claims 1-4, wherein said second treatment cycle is a second treatment cycle subsequent to the first treatment cycle.

6. The regimen according to any one of claims 1-5, wherein said second dose is administered every two weeks during the second treatment cycle.

7. The regimen according to any one of claims 1-6, wherein said second dose is 8 mg/kg to 15 mg/kg, preferably 8 mg/kg, 12 mg/kg or 15 mg/kg, most preferably 8 mg/kg.

8. The regimen according to any one of claims 1-7, wherein the multiple-variable dosage regimen can also be combined with other regimen.

9. The regimen according to any one of claims 1-8, wherein said cancer with high EGFR expression is colorectal cancer, head and neck cancer, lung cancer, breast cancer, esophageal cancer, gastric cancer, intestinal cancer, gastroesophageal junction cancer, liver cancer, pancreatic cancer, cholangiocarcinoma, gallbladder carcinoma, kidney cancer, or ovarian cancer.

10. The regimen according to any one of claims 1-9, wherein said subject suffers from metastatic colorectal cancer and has failed treatment with fluorouracil, oxaliplatin, and irinotecan;
Wherein, the multiple-variable dosage regimen includes dose of 6 mg/kg once a week for the first 6 weeks; subsequent dose of 8 mg/kg once every two weeks for maintenance treatment until disease progression or intolerable toxicity occurs.

11. The regimen according to any one of claims 1-9, wherein said subject suffers from recurrent and/or metastatic head and neck squamous cell carcinoma and has failed platinum-based drugs treatment;
Wherein, the multiple-variable dosage regimen includes dose of 6 mg/kg once a week for the first 6 weeks; subsequent dose of 8 mg/kg once every two weeks for maintenance treatment until disease progression or intolerable toxicity occurs.

12. The regimen according to any one of claims 1-9, wherein said subject suffers from recurrent and/or metastatic head and neck squamous cell carcinoma and has not received systemic treatment previously;
Wherein, the multiple-variable dosage regimen includes dose of 6 mg/kg once a week for the first 6 weeks; subsequent dose of 8 mg/kg once every two weeks for maintenance treatment;
Wherein, the multiple-variable dosage regimen is combined with the PF regimen, wherein said PF regimen is a cisplatin combined 5-fluorouracil treatment regimen, and the cisplatin dosage regimen is dosing on the first day with a dose of 75mg/m²; the 5-fluorouracil dosage regimen is dosing for 5 consecutive days with a dose of 750mg/m²;
Wherein, the total dosing cycle is up to 6 cycles of treatment wherein 3 weeks is 1 cycle, until disease progression or intolerable toxicity occurs.

13. The regimen according to any one of claims 1-9, wherein said subject suffers from recurrent or metastatic triple-negative breast cancer that has received at least first-line systemic treatment previously;
Wherein, the multiple-variable dosage regimen includes dose of 6 mg/kg once a week for the first 6 weeks; subsequent dose of 8 mg/kg once every two weeks for maintenance treatment until disease progression or intolerable toxicity occurs.

14. The regimen according to any one of claims 1-9, wherein said subject suffers from advanced esophageal squamous cell carcinoma that has failed platinum-based, taxane-based, or fluorouracil-based therapy previously;
Wherein, the multiple-variable dosage regimen includes dose of 6 mg/kg once a week for the first 6 weeks; subsequent dose of 8 mg/kg once every two weeks for maintenance treatment until disease progression or intolerable toxicity occurs.

15. The regimen according to any one of claims 1-9, wherein said subject suffers from locally advanced or metastatic squamous non-small cell lung cancer that has failed treatment with at least two chemotherapy regimens previously;
Wherein, the multiple-variable dosage regimen includes: dose of 9 mg/kg once a week for the first 6 weeks; subsequent dose of 12 mg/kg once every two weeks for maintenance treatment until disease progression or intolerable toxicity; or dose of 12mg/kg once a week for the first 6 weeks; subsequent dose of 15mg/kg once every two weeks for maintenance treatment until disease progression, or intolerable toxicity occurs.

16. The regimen according to any one of claims 1-9, wherein said subject suffers from an advanced solid tumor that has failed standard treatments previously;
Wherein, the multiple-variable dosage includes dose of 6 mg/kg once a week for the first 6 weeks; subsequent dose of 8 mg/kg once every two weeks for maintenance treatment until disease progression or intolerable toxicity occurs;
Wherein, the solid tumor is selected from one or more of gastric cancer, gastroesophageal junction cancer, liver cancer, pancreatic cancer, cholangiocarcinoma, gallbladder carcinoma, kidney cancer, and ovarian cancer.

17. The regimen according to any one of claims 1-16, wherein said anti-EGFR antibody or antigen-binding fragment thereof comprises a light chain variable region having a light chain CDR1 region having the amino acid sequence shown in SEQ ID NO: 1, a light chain CDR2 region having the amino acid sequence shown in SEQ ID NO: 2, and a light chain CDR3 region having the amino acid sequence shown in SEQ ID NO: 3; and a heavy chain variable region having a heavy chain CDR1 region having the amino acid sequence shown in SEQ ID NO: 4, a heavy chain CDR2 region having the amino acid sequence shown in SEQ ID NO: 5 and a heavy chain CDR3 region having the amino acid sequence shown in SEQ ID NO:6.

18. The regimen according to claim 17, wherein said anti-EGFR antibody or antigen-binding fragment thereof comprises a light chain variable region having the amino acid sequence shown in SEQ ID NO: 7 or the amino acid sequence having at least 90%, 92%, 95%, 98%, or 99% sequence identity therewith, and a heavy chain variable region having the amino acid sequence shown in SEQ ID NO: 8 or the amino acid sequence having at least 90%, 92%, 95%, 98%, or 99% sequence identity therewith.

19. The regimen according to claim 17 or claim 18, wherein said anti-EGFR antibody is an IgGlK type monoclonal antibody.

20. The regimen according to claim 19, wherein said anti-EGFR antibody comprises a light chain constant region having the amino acid sequence shown in SEQ ID NO: 9 or the amino acid sequence having at least 90%, 92%, 95%, 98%, or 99% sequence identity therewith., and a heavy chain constant region having the amino acid sequence shown in SEQ ID NO: 10 or the amino acid sequence having at least 90%, 92%, 95%, 98%, or 99% sequence identity therewith.

21. The regimen according to claim 20, the binding affinity of said anti-EGFR antibody to the EGFR ECD recombinant protein KD = 0.5×10⁻¹⁰M.

22. The regimen according to claim 18 or claim 21, wherein said anti-EGFR antibody or antigen-binding fragment thereof is N6-3 or antigen-binding fragment thereof.
